# EUROPEAN PATENT SPECIFICATION

(11) **EP 2 802 226 B1**
(45) Date of publication and mention of the grant of the patent: **27.12.2017**
(21) Application number: 13702662.1
(22) Date of filing: 11.01.2013
(51) Int. Cl.: A24F 47/00

(54) **SMOKING ARTICLE**
RAUCHARTIKEL
ARTICLE À FUMER

(30) Priority: 13.01.2012 GB 201200558
(43) Date of publication of application: 19.11.2014
(60) Divisional application: 17201802.0
(73) Proprietor: British American Tobacco (Investments) Limited, London WC2R 3LA (GB)
(72) Inventor: WOODCOCK, Dominic, London WC2R 3LA (GB)
(74) Representative: EIP
(86) International application number: PCT/GB2013/050050
(87) International publication number: WO 2013/104914

(56) References cited:
- EP-A2- 0 380 324
- WO-A1-2011/139730
- US-A1- 2004 173 229
- US-A1- 2011 041 861

## Description

This invention relates to an insulating material for use in a Heat-Not-Burn (HNB) smoking article.

HNB technology generates an aerosol by heating, rather than burning, tobacco and/or other smoking materials. Because the tobacco is not burned, there is a reduced amount of sidestream smoke and combustion and pyrolysis products. The odour, stain and ash of conventional cigarettes are also mostly eliminated. Nicotine and flavour, however, which are sought by the smoker in the mainstream smoke, are retained.

Examples of HNB cigarettes are disclosed in International Publication No. WO 2007/108878. In such known HNB cigarettes, a combustible carbonaceous fuel element is provided at a distal (i.e. non-buccal) end of the cigarette, and it is this fuel element that provides the heat to vaporise humectants and flavours from an adjacent tobacco section. The vapour, containing nicotine and flavour, may be inhaled by the smoker drawing on the buccal end of the cigarette.

In use, the fuel element may generate very high temperatures, for example up to around 800°C. In order to reduce the peripheral temperature of the cigarette and prevent an adjacent tobacco section from igniting, the fuel element may be wrapped in insulation material composed of glass fibres.

In some HNB cigarettes, a carbon monoxide (CO) catalyst may be applied directly to the fuel element, in order to reduce the yield of CO from the cigarette during the smoking experience. However, the amount of catalyst which may be added to the fuel element has to be sufficiently low in order to not adversely affect the combustion properties of the fuel element. As such, the benefit of the reduced yield of CO has been limited in this form.

US 2004/0173229 describes smoking articles comprising ultrafine particles which may catalyse the oxidation of carbon monoxide. It describes a combustible material component of a smoking article which may comprise ultrafine particles. In other embodiments, ultrafine particles are incorporated into the smoking article in proximity to a combustible material component.

US 2011/0041861 describes insulation materials for smoking articles which include non-glass components.

EP 0380324 concerns smoking articles such as cigarettes and has as its objective the provision of a smoking article in which sidestream smoke is substantially reduced or even eliminated by a means which involves substantially quenching the combustion of the smoking material between puffs and reigniting said smoking material at the instigation of puffing.

WO 2011/139730 relates to smoking articles and describes insulative materials that may include glass or non-glass fibres and foamed monoliths including metals, ceramic and ceramic-metal composites.

According to a first aspect of the invention, there is provided an insulating material for use in an HNB smoking article according to claim 1, wherein the insulating material comprises a sol-gel or aerogel, and wherein the material comprises a CO catalyst, but does not comprise glass fibre.

According to a second aspect of the invention, there is provided a method of making an insulating material according to the first aspect.

According to a third aspect of the invention, there is provided an HNB smoking article comprising an insulating material of the first aspect.

According to a fourth aspect of the invention, there is provided a method for reducing the amount of CO present in an aerosol produced by an HNB smoking article, the method using an insulating material of the first aspect.

As used herein, the term "aerosol" can include vapours, gases, particles and the like, both visible and invisible, and especially those components perceived by the user to be "smoke-like", generated by action of the heat from a burning fuel element upon substances contained within an aerosol-generating substance, or elsewhere in a smoking article.

Preferred embodiments of the present invention will now be described with reference to the accompanying drawings, in which:
Figure 1 is a perspective view of an existing HNB product; and
Figure 2 is a cross-sectional view of the distal (i.e. non-buccal) end of a smoking article according to one embodiment of the present invention.

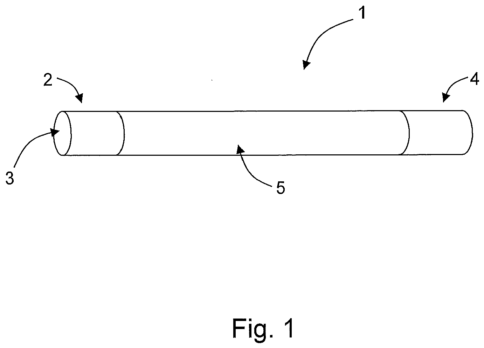

Figure 1 is an illustration of a known HNB product, such as a cigarette **1**, having a fuel element **3** housed in a distal end **2** of the cigarette, and a filter section or mouthpiece **4** at the buccal (mouth) end of the cigarette. The fuel element **3** is circumscribed by an insulating mantel of glass fibres (not shown). Between the filter section or mouthpiece **4** and the fuel element **3** are one or more tobacco sections **5** which are arranged to generate an aerosol for delivery to the consumer. The aerosol subsequently passes through the filter section or mouthpiece **4** to the mouth of the user. The cigarette **1** is circumscribed by a suitable wrapping material, such as paper.

As described above, it is possible to apply a CO catalyst directly to the fuel element of HNB cigarettes in order to reduce the yield of CO from the cigarette during the smoking experience. For example, metals, metal halides and/or metal oxides may be incorporated into the fuel element **3**, which catalyse the conversion of CO to carbon dioxide (CO₂). However, the quantity of catalyst which may be added to the fuel element has to be sufficiently low in order to maintain the combustion properties of the fuel element. Therefore, the effect of the CO catalyst in reducing the amount of CO in the produced vapour is limited.

The present inventor has appreciated a way in which a greater quantity of a CO catalyst can be added to an HNB device.

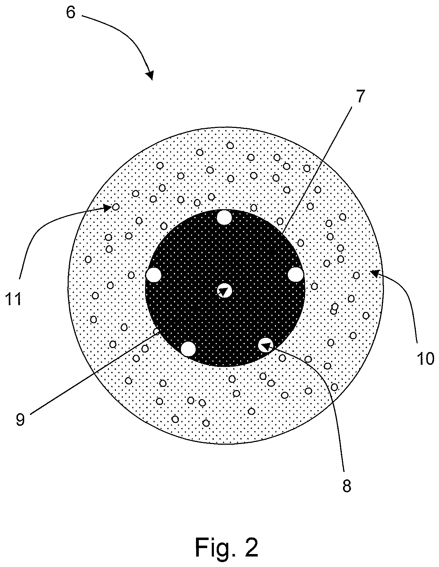

Figure 2 shows a cross-sectional view of the distal end of an exemplary HNB smoking article **6** according to one aspect of the present invention. The HNB smoking article **6** may be a cigarette, cigar or cigarillo, the physical dimensions of which closely approximate those of the conventional forms.

As shown in Figure 2, the fuel element 7 consists of a rod of combustible carbonaceous material, such as carbon, that serves as the high temperature heat source for generating an aerosol. The rod has a plurality of shallow longitudinal grooves **8** and a thin central axial channel **9**. These elements facilitate heating the air that passes over the hot surface of the rod as the smoker draws on the cigarette during the smoking experience.

The fuel element **7** is surrounded by insulating material **10** comprising particles of a CO catalyst **11**. The insulating material **10** may be co-extensive with the fuel element **7** (i.e. the ends of the fuel element **7** may be flush with the ends of the insulating material **10**). Alternatively, the insulating material **10** maybe shorter than the fuel element **7**, or it may be longer than the fuel element **7**. The fuel element **7** may be approximately 2 mm in diameter and approximately 12 mm in length. Advantageously, the insulating material **10** is at least about 0.5 mm thick, preferably at least about 1 mm thick, and more preferably between about 1.5 to 2 mm thick. The insulating material **10** may be formed from a sol-gel, aerogel, foamed ceramic or such like. The insulating material **10** is not formed from glass fibres or filaments. The insulating material **10** serves to buffer the heat between the fuel element **7** and periphery, and also between the fuel element **7** and adjacent tobacco section (not shown). It also holds the fuel element **7** in place.

Any catalyst capable of facilitating the oxidation of CO to CO2 may be used. Suitable CO catalysts include metals, metal salts, metal oxides or combinations thereof. Optionally, the metal may be aluminium, copper, iron, nickel, zinc, zirconium, a transition metal, lanthanide metal or actinide metal. The metal may be a precious metal such as ruthenium, rhodium, palladium, osmium, iridium, platinum, silver or gold. Exemplary metal salts include citrates, nitrates, sulphates, cyanates, hydrides, amides, thiolates, carbonates, halides and such like. Preferred metal oxides are iron oxide, copper oxide, zinc oxide and cerium oxide, the latter being particularly preferred. Preferred metal halides are platinum chloride and palladium chloride. Mixtures of such catalysts may be used.

The catalyst may be placed on a support or carrier made of graphite, activated carbon, copper oxide, alumina or titania, for example. The carrier may be uniformly coated with the catalyst, the loading being from about 0.1% to about 10%, based on the total dry weight of the coated support or carrier.

The catalyst may be provided in the form of coarse, fine or ultrafine particles. Coarse particles are particles having a diameter of about 2.5 µm to about 200 µm. Fine particles are particles having a diameter of about 100 nm to about 2.5 µm. Ultrafine particles are particles having a diameter of less than about 100 nm. Typically, the particles have an average particle size of between about 1 nm and 100 µm, for example, between about 10 nm to about 10 µm. Particles of catalyst may be obtained commercially.

One aspect of the present invention relates to adding a CO catalyst to the insulating material as it is manufactured. The manufacture of insulating materials composed of sol-gels, aerogels or foamed ceramics is known. The catalyst may be added to the insulating material in dry powder form, or in solution or colloidal form, by direct mixing with the insulating material mix. The catalyst may be added at the start of and/or during the mixing process. In another embodiment, the catalyst may be co-extruded with the insulating material. Alternatively, the insulating material may be manufactured prior to incorporation of the catalyst. For example, the pre-formed insulating material can be coated, sprayed or immersed in a catalyst provided in a suitable form. The catalyst can be applied to the insulating material just prior to inclusion of the latter in an HNB product. The catalyst can be randomly or essentially homogenously distributed within the insulating material.

The catalyst may be added to the insulating material in any suitable amount. For example, the amount can range from about 0.1 mg to about 500 mg in an HNB product of the present invention. Generally, at least 1 mg, and often at least 5 mg, catalyst will be present in an HNB product of the present invention. The catalyst may be present in an HNB product of the present invention in an amount of from about 5 mg to about 20 mg. However, the amount of catalyst present in the HNB product may exceed about 20 mg, about 50 mg, about 100 mg, about 200 mg, about 300 mg or even about 400 mg. Once the catalyst and insulating material are combined, the catalyst may comprise up to 75% (w/w) of the resulting mixture, for example, it may comprise up to 60%, 50%, 40%, 30%, 20% or 10% (w/w) of the resulting mixture. As a minimum, the mixture may comprise at least 10%, 20%, 30%, 40%, 50% or 60% (w/w) catalyst.

The catalyst is preferably added to the insulating material in an active form, that is to say, it is preferably not added as an inactive precursor. Thus, the catalyst preferably does not depend upon the heat generated by the fuel element in the HNB product, when in use, for its activation.

The insulating material may be multi-layered. The CO catalyst may be added to one, two, three or more layers. For example, the innermost layer may comprise a CO catalyst, while the remaining layers do not. Or, each layer may comprise a different CO catalyst.

Once formed, a suitable amount of insulating material comprising the catalyst may be wrapped around a suitably sized fuel element. Or, the insulating material and fuel element may be co-extruded in a continuous extrusion process, the extruded rod being divided into appropriately-sized shorter segments thereafter. Suitable procedures will be known to the skilled person.

The insulated fuel element can then be juxtaposed to a rod of tobacco material. Any tobacco material may be used. The tobacco rod may contain one or more different tobacco sections. For example, a first section may comprise shredded reconstituted tobacco containing a suitable aerosol-forming substance (such as glycerine, triethylene glycol, propylene glycol or other polyhydric alcohol), and a second section may contain blended tobacco. A heated aerosol generated in the first section may thus be directed downstream over the second section, which delivers tobacco flavour and nicotine to the heated aerosol. The aerosol will thus contain the taste and flavours of tobacco without the numerous combustion products produced by a conventional cigarette. At least one of the tobacco sections may be foil-wrapped, to aid the downstream passage of the aerosol.

The tobacco rod can be juxtaposed to a filter element or mouthpiece. If a filter is included, it may consist of a bundle of cellulose acetate fibres, paper or other suitable filtration material, as per conventional cigarettes.

Typically, a circumscribing outer overwrap is subsequently applied to the combined segments, to produce the finished HNB product.

Other cigarette components (for example, additives such as flavourants, humectants, binders and adhesives), component designs (for example, ventilated filters) and design configurations and formats are envisaged and included herein.

In use, the smoker lights the distal end of an HNB product of the invention, such that the fuel element begins to burn. An aerosol may be generated by this burning process, and/or the heat generated by the burning fuel element may be passed to an adjacent tobacco section by conduction, convection or such like, for an aerosol to be formed there. As the smoker draws on the HNB product, the hot aerosol is swept through one or more downstream tobacco sections, to extract the volatile components from the tobacco, without combustion or substantial pyrolysis. Volatalised components, such as flavour and nicotine, thus become entrained in the air that is drawn through the one or more tobacco segments. The aerosol subsequently passes through a filter section or mouthpiece at the buccal end of the HNB product, and into the mouth of the smoker. Thus, when smoked, the HNB product yields visible mainstream aerosol that resembles the mainstream smoke of a conventional cigarette.

By incorporating a CO catalyst into the insulating material around the combustible heat source of the HNB product, it is possible to reduce the yield of CO from the cigarette. Indeed, the CO catalysts catalyse the oxidation of CO in the hot air or aerosol as it passes over the fuel element and into the downstream section(s) of the HNB product, thereby reducing the amount of CO present in the combustion gases produced by burning of the fuel element. Advantageously, the amount of CO reduction achieved in an HNB product of the invention is greater than has traditionally been achieved with known HNB products.

Thus, also provided herein is a method for reducing the amount of CO present in an aerosol produced by an HNB smoking article. The method comprises incorporating a CO catalyst into the insulating material that circumscribes the fuel element in the HNB smoking article. The insulating material and catalyst are as described herein.

The inventor has appreciated that glass fibre is not likely to be a good material for insulating the fuel element in the presence of a CO catalyst, as the inclusion of other materials within the glass fibre would change its structural properties.

However, CO catalysts are more effective when they are placed in close proximity to the fuel element because the catalysts are most efficient at high temperatures (above approximately 200 °C). Indeed, it has been found that CO catalysts are less effective if used in other parts of the smoking article away from the fuel element.

A further advantage of placing the CO catalyst within the insulating layer is that the catalyst will not be destroyed by the combustion process, as will likely be the case for catalysts added to a fuel cell as described in WO 2007/108878. Efficiency of the catalyst in oxidising CO is therefore maintained throughout the smoking experience.

The insulating materials used in the present invention, comprising a sol-gel and/or aerogel and optionally foamed ceramics, may additionally lower the peripheral temperature of the smoking article beyond that traditionally achieved in known HNB products. Traditional insulating materials, such as glass fibre, allow the peripheral temperature to reach 400 °C or so. The present invention permits a lower peripheral temperature than this to be achieved, which may reduce the associated safety risks.

Smoking articles of the present invention can be packaged for distribution, sale and use.

Above is described what are believed to be the preferred embodiments of the invention. However, those skilled in the art will recognise that changes and modifications may be made without departing from the scope of the invention.

## Claims

1. An insulating material for use in a Heat-Not-Burn (HNB) smoking article, wherein the insulating material comprises a sol-gel or aerogel, and wherein the material comprises a carbon monoxide (CO) catalyst, but does not comprise glass fibre.

2. An insulating material as claimed in claim 1, wherein the CO catalyst is cerium oxide, a metal halide or a precious metal.

3. An insulating material as claimed in any one of claims 1-2, wherein the insulating material comprises at least 200 mg of the CO catalyst.

4. A method of making an insulating material as claimed in any one of claims 1-3.

5. A method as claimed in claim 4, comprising incorporating the CO catalyst into the insulating material during manufacture of the insulating material.

6. A method as claimed in claim 5, wherein the CO catalyst is incorporated in an active form.

7. An HNB smoking article comprising an insulating material as claimed in any one of claims 1-3.

8. An HNB smoking article as claimed in claim 7, wherein the insulating material circumscribes a combustible heat source of the HNB smoking article.

9. A method for reducing the amount of CO present in an aerosol produced by an HNB smoking article, the method using an insulating material as claimed in any one of claims 1-3.

10. A method as claimed in claim 9, wherein the CO catalyst is not destroyed during use of the HNB smoking article.

## Patentansprüche

1. Isoliermaterial zur Verwendung in einem Heat-Non-Burn (HNB)-Rauchartikel, wobei das Isoliermaterial ein Sol-Gel oder Aerogel umfasst und wobei das Material einen Kohlenmonoxid (CO) -Katalysator umfasst, aber keine Glasfaser umfasst.

2. Isoliermaterial nach Anspruch 1, wobei der CO-Katalysator Ceroxid, ein Metallhalogenid oder ein Edelmetall ist.

3. Isoliermaterial nach einem der Ansprüche 1 bis 2, wobei das Isoliermaterial zumindest 200 mg des CO-Katalysators umfasst.

4. Verfahren zum Herstellen eines Isoliermaterials nach einem der Ansprüche 1 bis 3.

5. Verfahren nach Anspruch 4, welches das Integrieren des CO-Katalysators in dem Isoliermaterial während der Herstellung des Isoliermaterials umfasst.

6. Verfahren nach Anspruch 5, wobei der CO-Katalysator in einer aktiven Form integriert ist.

7. HNB-Rauchartikel, welcher ein Isoliermaterial nach einem der Ansprüche 1 bis 3 umfasst.

8. HNB-Rauchartikel nach Anspruch 7, wobei das Isoliermaterial eine brennbare Wärmequelle des HNB-Rauchartikels abgrenzt.

9. Verfahren zur Verringerung der Menge an CO, welches in einem Aerosol vorhanden ist, das durch einen HNB-Rauchartikel erzeugt wird, wobei das Verfahren ein Isoliermaterial nach einem der Ansprüche 1 bis 3 verwendet.

10. Verfahren nach Anspruch 9, wobei der CO-Katalysator bei der Verwendung des HNB-Rauchartikels nicht zerstört wird.

## Revendications

1. Matériau isolant destiné à être utilisé dans un article à fumer à chaleur sans brûlure (HNB), le matériau isolant comprenant un sol-gel ou un aérogel, et le matériau comprenant un catalyseur de monoxyde de carbone (CO), mais ne comprend pas de fibres de verre.

2. Matériau isolant selon la revendication 1, dans lequel le catalyseur de CO est l'oxyde de cérium, un halogénure métallique ou un métal précieux.

3. Matériau isolant selon l'une quelconque des revendications 1 et 2, le matériau isolant comprenant au moins 200 mg du catalyseur de CO.

4. Procédé de fabrication d'un matériau isolant selon l'une quelconque des revendications 1 à 3.

5. Procédé selon la revendication 4, comprenant l'incorporation du catalyseur de CO dans le matériau isolant durant la fabrication du matériau isolant.

6. Procédé selon la revendication 5, dans lequel le catalyseur de CO est incorporé sous une forme active.

7. Article à fumer HNB comprenant un matériau isolant selon l'une quelconque des revendications 1 à 3.

8. Article à fumer HNB selon la revendication 7, dans lequel le matériau isolant circonscrit une source de chaleur de combustible de l'article à fumer NHB.

9. Procédé de réduction de la quantité de CO présente dans un aérosol produit par un article à fumer HNB, le procédé utilisant un matériau isolant selon l'une quelconque des revendications 1 à 3.

10. Procédé selon la revendication 9, dans lequel le catalyseur de CO n'est pas détruit durant l'utilisation de l'article à fumer NHB.
